# EUROPÄISCHE PATENTSCHRIFT

(11) **EP 0 930 929 B1**
(45) Veröffentlichungstag und Bekanntmachung des Hinweises auf die Patenterteilung: **22.08.2001**
(21) Anmeldenummer: 97947005.1
(22) Anmeldetag: 07.10.1997
(51) Int. Cl.: B01D 53/84, B01D 53/85, C12N 1/36

(54) **MIKROBIOLOGISCHES VERFAHREN ZUR BESEITIGUNG VON HALOGENIERTEN ETHENEN**
MICROBIOLOGICAL METHOD FOR ELIMINATING HALOGENATED ETHENES
PROCEDE MICROBIOLOGIQUE D'ELIMINATION D'ETHYLENES HALOGENES

(30) Priorität: 07.10.1996 DE 19641268; 07.03.1997 DE 19709453
(43) Veröffentlichungstag der Anmeldung: 28.07.1999
(73) Patentinhaber: FRAUNHOFER-GESELLSCHAFT ZUR FÖRDERUNG DER ANGEWANDTEN FORSCHUNG E.V., 80636 München (DE)
(72) Erfinder: KOZIOLLEK, Petra, D-70569 Stuttgart (DE); BRYNIOK, Dieter, D-72144 Dusslingen (DE); KNACKMUSS, Hans-Joachim, D-71229 Leonberg (DE)
(74) Vertreter: Pfenning, Meinig & Partner
(86) Internationale Anmeldenummer: DE9702313
(87) Internationale Veröffentlichungsnummer: WO9815338

(56) Entgegenhaltungen:
- EP-A- 0 336 718
- EP-B- 0 447 862
- DE-A- 3 326 057
- "Untersuchungen zum mikrobiellen Abbau chlorsubstituierter Ethene", Dissertation Jens Ewers, 1991, Universität Stuttgart

## Beschreibung

Die Erfindung betrifft ein Verfahren zur mikrobiologischen Beseitigung von halogenierten Ethenen.

Halogenierte Ethene fanden in der Vergangenheit unter anderem als Entfettungsmittel in der metallverarbeitenden Industrie und bei der Textilreinigung weitverbreitete Anwendung. Inzwischen sind diese gesundheitsgefährdenden Stoffe vielerorts in Grundwasser und Boden nachweisbar.

Die Behandlung von Schadensfällen erfolgt vorwiegend durch Bodenluftansaugung bzw. durch Strippen des Grundwasserleiters mit Luft. Die mit flüchtigen Komponenten beladene Abluft wird anschließend über Aktivkohlefilter geleitet. Dabei werden die Schadstoffe durch Adsorption an die Aktivkohle aus dem Abluftstrom entfernt. Die beladene Aktivkohle kann regeneriert und erneut zur Reinigung der Abluftströme eingesetzt werden. Ist eine Regeneration der Aktivkohle nicht wirtschaftlich, wie im Falle der Reinigung niedrig beladener Abluftströme, wird die Aktivkohle zusammen mit den adsorbierten Schadstoffen verbrannt.

Bei der Verbrennung von CKW-beladener Aktivkohle können polychlorierte Dioxine entstehen. Die "17. Verordnung zum Bundesimmisionsschutzgesetz" schreibt daher für die thermische Abfallbehandlung einen Dioxin-Grenzwert von 0,1 ng/m³ Abluft vor. Die Regeneration von Aktivkohle ist dann unwirtschaftlich, wenn in dem zu reinigenden Abluftstrom nur geringe Konzentrationen an den zu entfernenden Schadstoffen vorhanden sind und deshalb die Aktivkohle aufgrund der Adsorptionsthermen nur gering beladen wird. Bei geringen Schadstoffkonzentrationen in der zu reinigenden Abluft sind aufgrund des niedrigen Beladungsspielraumes große Mengen an Aktivkohle erforderlich. Die thermische Entsorgung dieser Aktivkohle beeinflußt im Falle einer Boden- und Grundwassersanierung als wesentlicher Kostenfaktor erheblich die Wirtschaftlichkeit des Verfahrens.

In der Praxis wird oft der Abluftstrom nur in der Anfangsphase der Sanierung bei hohen Schadstoffkonzentrationen mit Aktivkohlefiltern gereinigt. Sinkt die Schadstoffkonzentration im Laufe des Sanierungsverfahrens unter die gesetzlich vorgeschriebenen Grenzwerte der TA Luft, wie dies bei der Bodenluftabsaugung üblich ist, so wird die Abluft ungereinigt an die Atmosphäre abgegeben. Diese Umverteilung der Schadstoffe von Boden, Grundwasser und Bodenluft in die Atmosphäre ist aus ökologischen Gründen fragwürdig.

Boden und vor allem Grundwasser sind hohe Schutzgüter. Aus umweltpolitischen Erwägungen ist man daher bestrebt, CKW-Schadstoffe dauerhaft zu sanieren, ohne eine Umverteilung der CKW in die Atmosphäre zu bewirken. Die Reinigung niedrig beladener Abluftströme aus der Bodenluftabsaugung ist jedoch sehr kostenintensiv, da große Mengen niedrigbeladener Aktivkohle anfallen, die nicht wirtschaftlich regeneriert werden können und unter hohen Kosten thermisch entsorgt werden müssen.

Unter anaeroben Bedingungen entsteht bei durch Tetrachlorethen (PCE) und Trichlorethen (TCE) verurachten Schadensfällen infolge der Aktivität der autochthonen Mikroflora des Bodens Vinylchlorid (VC), welches ein besonderes Problem darstellt. Zum einen ist es bekanntermaßen karzinogen und teratogen, zum anderen adsorbiert es nur in äußerst geringen technisch nicht relevanten Mengen an Aktivkohle. CKW-Schadensfälle mit höheren VC-Konzentrationen gelten daher als mit den beschriebenen Methoden nach dem derzeitigen Stand der Technik nicht sanierbar.

Als kostengünstige Alternative zur Reinigung von kontaminiertem Boden, Grundwasser und Bodenluft bieten sich aus diesen Gründen biologische Verfahren an. Eine Möglichkeit besteht beispielsweise darin, Methan in das Erdreich zu injizieren, um dort Methan oxidierende (methylotrophe) Bakterien anzureichern und zu aktivieren.

Die biologische Abbaurate ist abhängig von der Anzahl der Chlorsubstituenten und der An- bzw. Abwesenheit von Sauerstoff. Hochchlorierte CKW, insbesondere PCE, werden ausschließlich unter anaeroben Bedingungen, niedrig chlorierte CKW dagegen vorwiegend unter aeroben Bedingungen biologisch abgebaut. Methan oxidierende (methylotrophe) und Aromaten abbauende Bakterien sind beispielsweise in der Lage, TCE zu mineralisieren, also zu Kohlendioxid, Wasser und Chlorid umzuwandeln. Der Intitialangriff erfolgt durch eine Oxygenase. Durch die Oxygenase-Reaktion wird jedoch ein hochreaktives Epoxid gebildet, das die Zelle schädigt und desaktiviert. Die Schädigung erfolgt durch Alkylierung von Zellproteinen. Der Stamm Pseudomonas Putida F1 wird beispielsweise sehr schnell durch den TCE-Umsatz desaktiviert. Die Oxygenaseaktivität verringert sich innerhalb von 20 Minuten auf 2 % der Anfangsaktivität.

In der EP-B1-0 447 862 wird ein Verfahren zur biologischen Reinigung von mit halogenierten Ethenen und/oder halogenierten Butadienen kontaminierten Gasströmen, insbesondere aus der Bodenluft, beschrieben. Bei diesem Verfahren werden teure Wachstumssubstrate (Auxiliarsubstrate) wie Isopren und/oder Butadien eingesetzt. In der Praxis erwies sich dieses Verfahren als kostenintensiv, da die eingesetzten Bakterien zur Regeneration mit größeren Mengen Isopren versorgt werden müssen. Da es sich bei Isopren um eine relativ leicht abbaubare Verbindung handelt, ist das Risiko einer Fremdverkeimung durch nicht dehalogenierende Bakterien sehr hoch und kann nur durch aufwendige steriltechnische Maßnahmen vermieden werden, die sich erheblich auf die Betriebskosten auswirken.

In der EP-A-0 336 718 wird ein weiteres Verfahren zum mikrobiologischen Abbau von Trichlorethen mit Hilfe gentechnisch veränderter Mikroorganismen beschrieben. Vor Einsatz eines solchen Verfahrens zur Sanierung von Schadensfällen sind besondere Sicherheitsmaßnahmen zu treffen und langwierige Genehmigungsverfahren erforderlich. Außerdem sind Probleme bei der Akzeptanz durch die Bevölkerung nicht auszuschließen.

Die EP-A-3 326 057 offenbart den mikrobiologischen Abbau von Trichlorethenen unter anderem mit natürlich vorkommenden Mikroorganismen. Die abbauenden Zellen werden zunächst mit verschiedenen Verbindungen preadaptiert, wobei Toluolmonooxygenase-Gene induziert werden. Eine Zufuhr von Ethen zur Preadaptierung, Neubildung und Energieversorgung wird nicht beschrieben oder angeregt.

Keines der Verfahren ist geeignet, PCE abzubauen.

Ausgehend hiervon ist es die Aufgabe der vorliegenden Erfindung, ein Verfahren zur mikrobiellen Beseitigung/Entfernung von halogenierten Ethenen anzugeben, mit dem ein einfacher und vollständiger Abbau möglich ist.

Die Aufgabe wird durch die kennzeichnenden Merkmale des Anspruchs 1 gelöst. Die Unteransprüche zeigen vorteilhafte Weiterbildungen auf.

Erfindungsgemäß wird somit vorgeschlagen, Bakterien anzureichern, die Ethen als Kohlenstoff- und Energiequelle verwerten und die gleichzeitig in der Lage sind, halogenierte Ethene als Kohlenstoff, z.B. *c*DCE, zu mineralisieren. In einem Bioreaktor werden diese Bakterien mit Ethen als Kohlenstoff und Energiequelle versorgt. Die abzubauenden halogenierten Ethene werden in den Bioreaktor mit den präadaptierten Mikroorganismen geleitet. Dort werden die Chlorethene vollständig mineralisiert. Das Auxiliarsubstrat Ethen und die halogenierterten Ethen werden simultan und/oder konsekutiv abgebaut. Besonders geeignet ist das Verfahren für *cis*-1,2-Dichlorethen und/oder Vinylchlorid, das in Konzentrationen von bis zu 100 mg/l gereinigt werden kann.

Mit Hilfe des erfindungsgemäßen Verfahrens ist es möglich geworden, z.B. Grundwasser zu reinigen, das mit allen halogenierten Ethenen einschließlich PCE und VC kontaminiert ist. Dies ist auch bei gering belastetem Grundwasser und Erdreich möglich.

Bevorzugt wird das erfindungsgemäße Verfahren so durchgeführt, daß es in einer Kombination mit einer anaeroben Stufe angewendet wird. Als erste Stufe wird dabei die aus dem Stand der Technik bekannte Maßnahme ausgenutzt, daß anaerobe Bakterien und Mischkulturen in der Lage sind Chlorethene reduktiv zu dehalogenieren. Dabei wird PCE unter Freisetzung von Chlorid schrittweise zu TCE, cis-Dichlorethen (*c*DCE), VC und Ethen umgewandelt. Da die beiden ersten Dehalogenierungsschritte sehr effizient und schnell erfolgen, die Dehalogenierung von cDCE und insbesondere von VC jedoch in technisch nicht relevanten Raten, wird die vorstehend beschriebene anaerobe reduktive Dehalogenierung mit dem erfindungsgemäßen Verfahren kombiniert.

Ferner ist es mit Hilfe des erfindungsgemäßen Verfahrens möglich geworden, sowohl mit halogenierten Ethenen kontaminiertes Grundwasser als auch Boden und Bodenluft in situ zu reinigen. Die In-situ-Reinigung des kontaminierten Bodens kann erfolgen, indem das Grundwasser nach der Behandlung im Bioreaktor mit geeigneten Energie- und Kohlenstoffquellen, z.B. molekularem Wasserstoff, Kohlenhydraten, Alkoholen oder organischen Säuren, versetzt und in den Boden infiltriert wird. Damit wird ein anaerobes Milieu geschaffen und anaerobe, reduktiv dehalogenierende, autochthone Mikroflora aktiviert. Durch einen hydraulischen Kreislauf werden die hochchlorierten Ethene PCE und TCE im Boden zu cDCE dehalogeniert. Das Grundwasser wird abgepumpt und beispielsweise über der Geländeroberkante in Bioreaktoren behandelt. Die in der Bodenluft befindlichen Chlorethene lösen sich im reinfiltrierten Grundwasser. Verbliebene Reste der Chlorkohlenwasserstoffe können abgebaut werden, indem die Bodenluft zur Belüftung des aeroben Bioreaktors verwendet wird.

Überraschenderweise wurde gefunden, daß Ethen abbauende Bakterien in der Lage sein können, halogenierte Ethene abzubauen. Die beispielsweise von **Habets-Crützen et al.** (1984) Habets-Crützen, Brink, van Ginkel, de Bont, Tramper (1984): Production of Epoxides from Gaseous Alkenes by Resting-Cell Suspensions and Immobilized Cells of Alkene-Utilizing Bacteria. Appl. Microbiol. Biotechnol. 20: 245-250 aus der Arbeitsgruppe von de Bont früher beschriebenen Ethen abbauenden Bakterienstämme erreichten für Ethen-Abbauraten von 9 bis 50 nmol/·mg_{Protein}. **Hou et al.** (1983) Ewers (1991): Untersuchungen zum mikrobiellen Abbau chlorsubstituierter Ethene, Dissertation, Universität Stuttgart beschreiben eine Oxidation von Ethen durch Bakterien, die auf Propan gezüchtet wurden. Hier wurden 1,2 bis 43 nmol/min·mg_{Protein} gemessen. Auch **Ewers** (1991) Hou, Patel, Laskin, Barnabe, Barist (1983): Epoxidation of Short-Chain Alkenes by Resting-Cell Suspensions of Propane-Grown Bacteria. Appl. Environ. Microbiol. 46: 171-177 konnte Ethen abbauende Bakterienkulturen anreichern, machte jedoch keine Aussagen über die Abbauraten. Er untersuchte die Ethen abbauenden Kulturen auch auf Verwertung von Chlorethenen und Chloridfreisetzung, konnte jedoch nicht von positiven Ergebnissen berichten. Auch in **Heyer** (1976) Heyer (1976): Mikrobielle Verwertung von Äthylen. Zeitschr. f. allg. Mikrobiol. 16: 633-637 und den finden sich keine Hinweise auf einen Abbau von oder eine Chloridfreisetzung aus chlorierten Ethenen durch Ethen verwertende Bakterien.

Die dem erfindungsgemäßen Verfahren zugrunde liegenden Bakterienkulturen bauen Ethen in Raten bis zu 60 nmol/min·mg_{Protein} ab und sind in der Lage, halogenierte Ethene, wie z.B. cDCE, vollständig zu mineralisieren und dabei auch extrem hohe Konzentrationen dieser toxischen Verbindungen zu tolerieren. Die hohe Resistenz gegenüber halogenierten Ethenen beruht darauf, daß die beim oxidativen Abbau primär gebildeten Epoxide durch die Ethen verwertenden Bakterienstämme sehr wirksam entgiftet werden.

Ethen wird von den Mikroorganismen, die zur Verwertung dieses Substrats befähigt sind, im ersten Schritt der Abbausequenz durch eine Oxygenase-Reaktion zu Ethylenoxid oxidiert. Um eine Zellschädigung durch diese hochtoxische Verbindung, die auch zu Sterilisationszwecken eingesetzt wird, zu verhindern, sind diese Mikroorganismen auf ein hocheffizientes epoxidspaltendes Enzymsystem angewiesen, das Ethylenoxid entgiftet. Dieses Enzymsystem ist auch in der Lage, die beim primären Oxidationsschritt aus Chlorethenen entstandenen Epoxide zu spalten. Auf diese Weise können präadaptierte Mikroorganismen selbst hohe Konzentrationen von halogenierten Ethenen tolerieren und dehalogenieren.

Geeignete Bakterien zur Durchführung des erfindungsgemäßen Verfahrens erhält man durch Anreicherung mit Ethen als einziger Kohlenstoff- und Energiequelle gemäß den Fachleuten bekannten Techniken mit Proben aus geeigneten Standorten, die sowohl Ethen als auch Chlorethenen ausgesetzt waren. Bakterienkulturen, die auf diese Weise angereichert wurden, weisen folgende überraschende Eigenschaften auf:
- hohe Toleranz gegenüber halogenierten Ethenen, wie z.B. cDCE,
- hohe Abbauaktivität gegenüber halogenierten Ethenen, wie z.B. *c*DCE,
- Fähigkeit zum simultanen Abbau von halogenierten Ethenen und dem Auxiliarsubstrat Ethen,
- geringer Bedarf an Ethen als Auxiliarsubstrat,
- geringe Anfälligkeit für Fremdverkeimung bei Anwendung des erfindungsgemäßen Verfahrens.

Der CKW-Abbau durch die in EP-B1-0 447 862 beschriebenen Isopren und/oder Butadien abbauenden Bakterienstämme erfolgt nur in Abwesenheit geeigneter Kohlenstoff- und Energiequellen. Nach einigen Stunden des CKW-Abbaus läßt ihre Aktivität durch den allmählich auftretenden Energiemangel nach, so daß sie durch Zugabe von Isopren und/oder Butadien reaktiviert werden müssen. Im Gegensatz dazu sind die der Erfindung zugrunde liegenden Bakterienkulturen in der Lage, Chlorethen und Ethen simultan abzubauen, so daß sich verfahrenstechnich aufwendige Regenerationszyklen erübrigen. Der Abbau von Chlorethenen erfordert bei allen bisher in der Fach- und Patentliteratur beschriebenen Bakterienkulturen den Einsatz des jeweiligen geeigneten Auxiliarsubstrats im Überschuß. Überraschenderweise benötigen die der Erfindung zugrunde liegenden Ethen abbauenden Bakterienstämme lediglich eine - verglichen mit den halogenierten Ethenen - signifikant geringere Menge des Auxiliarsubstrats Ethen, um den Energiestoffwechsel und die Abbauaktivität gegenüber Chlorethenen aufrechtzuerhalten. Für das erfindungsgemäße Verfahren können also geringe Konzentrationen des Auxiliarsubstrats eingesetzt werden, wodurch sich die Wirtschaftlichkeit erhöht. Die Versorgung der Mikroorganismen mit Ethen erfolgt über die Gasphase. Die Konzentrationen sind so niedrig (unter 2,3 % (v/v)), daß kein explosives Gasgemisch entstehen kann, wodurch aufwendige Explosionsschutzmaßnahmen hinfällig werden.

Einzelne angereicherte Bakterienkulturen sind sogar über längere Zeiträume zum produktiven Abbau, also zur Verwertung von halogenierten Ethenen, wie z.B. cDCE, als einzige Energie- und Kohlenstoffquelle, befähigt.

Es zeigte sich, daß die der Erfindung zugrunde liegenden Ethen abbauenden Bakterienkulturen gegenüber Fremdverkeimung unempfindlich sind. Luftkeime oder Fremdkeime aus dem Wasser, die Ethen und/oder halogenierte Ethene unspezifisch oxidieren, bilden das hochtoxische Ethylenoxid oder Epoxide aus den halogenierten Ethenen, was ohne hocheffiziente epoxidspaltende Enzymsysteme zu schweren Zellschädigungen führt. Bei dem erfindungsgemäßen Verfahren sind also aufwendige und kostenintensive steriltechnische Maßnahmen zur Vermeidung einer Fremdverkeimung und zur Aufrechterhaltung des biologischen Abbauprozesses nicht notwendig.

Ein weiterer Vorteil des erfindungsgemäßen Verfahrens besteht darin, daß es sich bei Ethen um ein vergleichsweise preiswertes Auxiliarsubstrat handelt.

Im folgenden wird das erfindungsgemäße Verfahren anhand von Ausführungsbeispielen näher erläutert:

### Beispiel 1

### Anreicherung von geeigneten Mikroorganismen

Zur Anreicherung geeigneter Bakterien wurde Belebtschlamm aus der Betriebskläranlage eines Ethen und CKW produzierenden Industrieunternehmens als Inokulum eingesetzt. Als einziger Kohlenstoff- und Energiequelle wurde Ethen verwendet. Zur Anzucht der Flüssigkulturen wurde die von Dorn et al. (1974) Dorn, Hellwig, Reineke, Knackmuss (1974): Isolation and Characterization of a 3-Chlorobenzoate Degrading Pseudomonad. Arch. Microbiol. 99: 62-70 angegebene Mineralsalzlösung verwendet. Ethen wurde in den Konzentrationen zwischen 3 % und 20 % (jeweils v/v in der Gasphase) zugegeben. Die Inkubation erfolgte in Schüttelkolben mit Schikanen und einem Nennvolumen von 100 ml bzw. 500 ml, in denen sich 10 % des Nennvolumens an Mineralmedium befanden, bei 30 °C auf einen Rotationsschüttler bei 135 Upm. Das bakterielle Wachstum wurde durch Messung der optischen Dichte bei einer Wellenlänge von 546 nm in einem Photometer (DU 50, Beckmann Instruments Inc., UK) bestimmt. Die Isolierung der Bakterien erfolgte auf Agarplatten (Agar No. 1 Oxoid Ltd., London, UK) mit Mineralmedium. Ethen wurde hierbei über die Gasphase (1,9 % v/v) zudosiert.

### Beispiel 2

### Screening nach dehalogenierenden Mikroorganismen

Die Dehalogenierungsaktivität der angereicherten Bakterien wurde über die entstehende Chloridmenge bestimmt. Hierzu wurde der Chloridtest von Weightman et al. (1985) Weightman, Weightman, Slater (1985): Toxic effects of Chlorinated and Brominated Alkanoic Acids on *Pseudomonas putida* PP3: Selection at High Frequencies of Mutations in Genes Encoding Dehalogenases. Appl. Environ. Microbiol. 49: 1494-1501 für auf Platten gezüchtete Zellen bzw. ein von Ewers (1991) Hou, Patel, Laskin, Barnabe, Barist (1983): Epoxidation of Short-Chain Alkenes by Resting-Cell Suspensions of Propane-Grown Bacteria. Appl. Environ. Microbiol. 46: 171-177 modifizierter Test nach Weightman für Flüssigkulturen verwendet. Es wurde jeweils 1 mM cDCE bei diesem Test eingesetzt. Die Kulturen mit hohem Abbaupotential wurden am Silberniederschlag erkannt.

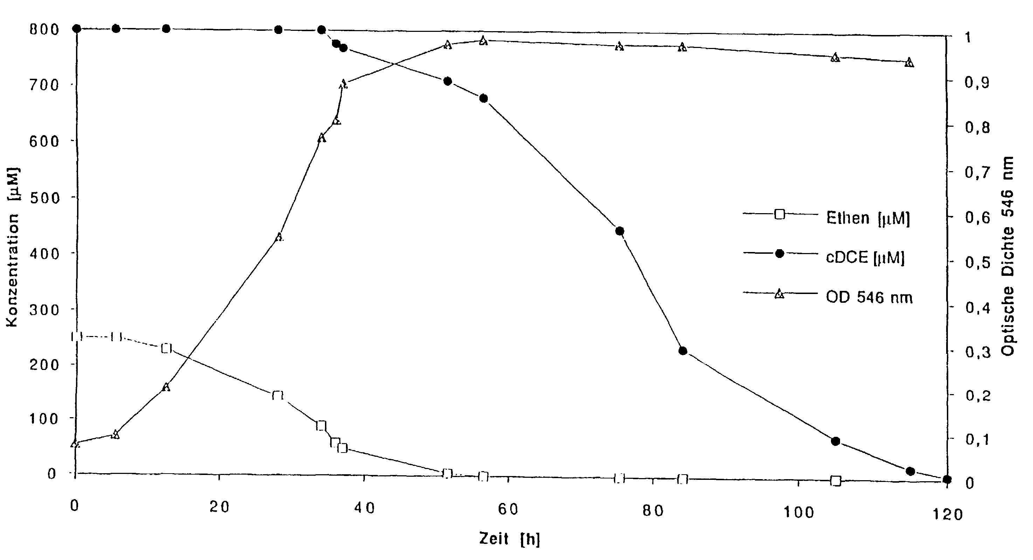

**Figur 1** zeigt das Wachstum einer Mischkultur auf 250 *µ*M Ethen in Gegenwart von 800 *µ* M *cis*-1,2-Dichlorethen in einer Batch-Kultur. Die Konzentrationen beziehen sich auf die Flüssigphase. Die Anzucht erfolgte in einem Schüttelkolben mit Schikanen und einem Nennvolumen von 500 ml. Die zu untersuchenden Proben wurden mit einer gasdichten Spritze durch ein Septum aus dem Gasraum des Kolbens entnommen. Die Detektion von Ethen und cDCE erfolgte mit Hilfe eines Gaschromatographen (Injektor-, Detektor- und Ofentemperatur 250 °C, 200 °C und 170 °C, FID, Plora PLOT U-Säule (25 m x 0,53 mm) mit He (30 kPa Säulenvordruck) als Trägergas). Die optische Dichte wurde bei 546 nm bestimmt. *Cis*-1,2-Dichlorethen wird am Ende der exponentialen Phase und während der stationären Phase abgebaut.

## Patentansprüche

1. Mikrobiologisches Verfahren zur Beseitigung von halogenierten Ethenen,
dadurch **gekennzeichnet,**
daß man mit Ethen als Kohlenstoff- und Energiequelle wachsende und halogenierte Ethene abbauende Bakterienstämme mit Ethen als Kohlenstoff- und Energiequelle zur Neubildung und zur Energieversorgung präadaptiert und die Bakterien dann in einem aeroben Bioreaktor simultan und/oder konsekutiv mit Ethen und den zu beseitigenden halogenierten Ethenen kontaktiert.

2. Verfahren nach Anspruch 1,
dadurch gekennzeichnet, daß Ethen als Kohlenstoff- und/oder Energiequelle über die Gasphase mit der Zuluft in einer Konzentration von unter 2,3 % (v/v) in den Bioreaktor eingeführt wird.

3. Verfahren nach Anspruch 1 oder 2,
dadurch gekennzeichnet, daß die Bakterienstämme im Bioreaktor mit einem zu reinigenden Wasser, enthaltend halogenierte Ethene, kontaktiert werden.

4. Verfahren nach Anspruch 3,
dadurch gekennzeichnet, daß kontanimiertes Grundwasser gereinigt wird.

5. Verfahren nach mindestens einem der Ansprüche 1 oder 2,
dadurch gekennzeichnet, daß man mit halogenierten Ethenen kontaminierte Luft zur Begasung des Bioreaktors einsetzt.

6. Verfahren nach Anspruch 5,
dadurch gekennzeichnet, daß man mit halogenierten Ethenen kontaminierte Bodenluft einsetzt.

7. Verfahren nach mindestens einem der Ansprüche 1 bis 6,
dadurch gekennzeichnet, daß vor der Reinigungsstufe im aeroben Bioreaktor eine anaerobe Stufe zur reduktiven Dehalogenierung der halogenierten Ethenen durchgeführt wird.

8. Verfahren nach mindestens einem der Ansprüche 1 bis 7,
dadurch gekennzeichnet, daß *cis*-1, 2-Dichlorethen und/oder Vinylchlorid enthaltendes Wasser und/oder Luft gereinigt wird.

9. Verfahren nach Anspruch 8,
dadurch gekennzeichnet, daß man Konzentrationen von *cis*-1,2-Dichlorethen und/oder Vinylchlorid bis zu 100 mg/l reinigt.

## Claims

1. Microbiological process for removing halogenated ethenes, characterized in that bacterial strains which are growing with ethene as carbon and energy source and break down halogenated ethenes are preadapted with ethene as carbon and energy source for new formation and for energy supply and the bacteria are then contacted simultaneously and/or consecutively with ethene and the halogenated ethenes to be removed in an aerobic bioreactor.

2. Process according to Claim 1, characterized in that ethene is introduced into the bioreactor as carbon source and/or energy source via the gas phase with the feed air in a concentration of below 2.3 % (v/v).

3. Process according to Claim 1 or 2, characterized in that the bacterial strains are contacted in the bioreactor with a water to be cleaned up which comprises halogenated ethenes.

4. Process according to Claim 3, characterized in that contaminated groundwater is cleaned up.

5. Process according to at least one of Claims 1 or 2, characterized in that air contaminated with halogenated ethenes is used for introducing gas into the bioreactor.

6. Process according to Claim 5, characterized in that halogenated-ethene-contaminated soil air is used.

7. Process according to at least one of Claims 1 to 6, characterized in that, prior to the cleaning stage in the aerobic bioreactor, an anaerobic stage for the reductive dehalogenation of the halogenated ethenes is carried out.

8. Process according to at least one of Claims 1 to 7, characterized in that *cis*-1,2-dichloroethene-containing and/or vinyl-chloride-containing water and/or air is cleaned up.

9. Process according to Claim 8, characterized in that concentrations of *cis*-1,2-dichloroethene and/or vinyl chloride up to 100 mg/l are cleaned up.

## Revendications

1. Procédé microbiologique pour l'élimination d'éthènes halogénés, caractérisé en ce qu'on préadapte des souches bactériennes croissant avec de l'éthène en tant que source de carbone et d'énergie et dégradant les éthènes halogénés, avec de l'éthène en tant que source de carbone et d'énergie pour la néoformation et pour l'apport d'énergie, et on met ensuite les bactéries en contact, dans un bioréacteur aérobie, simultanément et/ou consécutivement avec de l'éthène et les éthènes halogénés à éliminer.

2. Procédé selon la revendication 1, caractérisé en ce que l'éthène, en tant que source de carbone et/ou d'énergie, est introduit dans le bioréacteur par l'intermédiaire de la phase gazeuse avec l'air d'arrivée, à une concentration de moins de 2,3 % (v/v).

3. Procédé selon la revendication 1 ou 2, caractérisé en ce que les souches bactériennes dans le bioréacteur sont mises en contact avec une eau à épurer contenant des éthènes halogénés.

4. Procédé selon la revendication 3, caractérisé en ce que de l'eau souterraine contaminée est épurée.

5. Procédé selon au moins l'une des revendications 1 et 2, caractérisé en ce qu'on utilise pour l'aération du bioréacteur de l'air contaminé par des éthènes halogénés.

6. Procédé selon la revendication 5, caractérisé en ce qu'on utilise de l'air au sol contaminé par des éthènes halogénés.

7. Procédé selon au moins l'une des revendications 1 à 6, caractérisé en ce qu'avant l'étape d'épuration dans le bioréacteur aérobie, on effectue une étape anaérobie pour la déshalogénation réductrice des éthènes halogénés.

8. Procédé selon au moins l'une des revendications 1 à 7, caractérisé en ce qu'on épure de l'eau et/ou de l'air contenant du *cis*-1,2-dichloréthène et/ou du chlorure de vinyle.

9. Procédé selon la revendication 8, caractérisé en ce qu'on purifie des concentrations de *cis*-1,2-dichoréthène et/ou de chlorure de vinyle allant jusqu'à 100 mg/l.
